(19) Europäisches Patentamt
European Patent Office
Office européen des brevets

(11) Veröffentlichungsnummer: **0 301 375 B1**

# (12) EUROPÄISCHE PATENTSCHRIFT

(45) Veröffentlichungstag der Patentschrift: **18.03.92**

(51) Int. Cl.5: **C07C 47/546**, C07C 45/28, C07C 45/00, C11B 9/00, A61K 7/46

(21) Anmeldenummer: **88111578.6**

(22) Anmeldetag: **19.07.88**

## (54) Bicyclische Aldehyde.

(30) Priorität: **29.07.87 CH 2892/87**

(43) Veröffentlichungstag der Anmeldung:
**01.02.89 Patentblatt 89/05**

(45) Bekanntmachung des Hinweises auf die Patenterteilung:
**18.03.92 Patentblatt 92/12**

(84) Benannte Vertragsstaaten:
**CH DE ES FR GB IT LI NL**

(56) Entgegenhaltungen:
**DE-A- 1 468 755**
**FR-A- 1 146 023**

(73) Patentinhaber: **L. GIVAUDAN & CIE Société Anonyme**

**CH-1214 Vernier-Genève(CH)**

(72) Erfinder: **Gonzenbach, Hans-Ulrich, Dr.**
**61bis rue de Lyon**
**CH-1203 Genf(CH)**

(74) Vertreter: **Urech, Peter, Dr. et al**
**Grenzacherstrasse 124 Postfach 3255**
**CH-4002 Basel(CH)**

EP 0 301 375 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Riechstoffe. Es handelt sich dabei um die Verbindungen der Formel

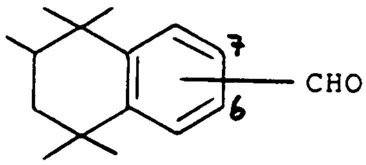

I

Die Formel I beinhaltet also das 1,1,2,4,4-Pentamethyl-6-formyl -1,2,3,4-tetrahydronaphthalin (Ia) und das 1,1,2,4,4-Pentamethyl-7-formyl-1,2,3,4-tetrahydronaphthalin (Ib).

Die Erfindung betrifft ferner ein Verfahren zur Herstellung der Verbindungen I.

Dieses Verfahren ist dadurch gekennzeichnet, dass man

a) eine Verbindung der Formel

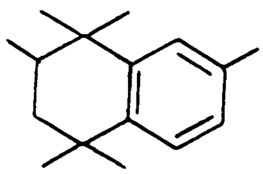

II

oxydiert, oder dass man

b) eine Verbindung der Formel

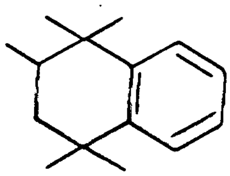

III

formyliert.

Die Oxydation der aromatischen Methylgruppe von II gemäss Verfahrensvariante a) kann in an sich bekannter Weise durchgeführt werden (siehe z.B. Organikum, Organisch-chemisches Grundpraktikum, 6. Auflage, VEB Deutscher Verlag der Wissenschaften, Berlin 1967; S. 335 seq.,).

Geeignete Oxydationsmittel sind demgemäss $CrO_3$, $SeO_2$, oder, insbesondere, $MnO_2/H_2SO_4$ oder $Mn^{III}$-Salze. Man arbeitet zweckmässigerweise in einem inerten Lösungsmittel wie Cyclohexan, Aethanol, Xylol, Naphthalin, Dioxan, etc. und bei Temperaturen von ca. 50 ° C bis ca. 100 ° C.

Die Formylierung von III gemäss Verfahrensvariante b) wird zweckmässigerweise mittels 1,1-Dichlordimethyläther nach A. Rieche bewerkstelligt, siehe diesbezüglich L.F. Fieser und M. Fieser, Reagents for Organic Synthesis, John Wiley and Sons, Inc. (1967), 220. Dabei fällt ein Gemisch von Ia und Ib an.

Die Verbindungen der Formel I zeichnen sich durch kräftige und sehr natürlich-warme Noten in Richtung Moschus mit strahlender, trockener Holznote in Richtung Patchouli aus. In Komposition kann sogar diese letztere Holznote überwiegend zum Tragen kommen, wobei gleichzeitig ausgesprochen harmonisierende Effekte beobachtet werden, wie diese üblicherweise nur mit ätherischen Oelen erzielt werden können. Solche Kompositionen zeichnen sich überdies durch eine starke Diffusion und stark verbesserte Substantivität aus.

Die Erfindung betrifft demgemäss auch die Verwendung der Verbindungen I als Riechstoffe.

Es sind zwar bereits Aldehyde sehr ähnlicher Struktur bekannt geworden, z.B. die Verbindung

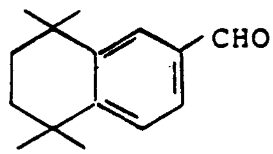

IV

aus J. Org. Chem. 28, 2248 (1963)(a) und aus The Givaudanian. September (1968)(b) bzw. die Verbindung

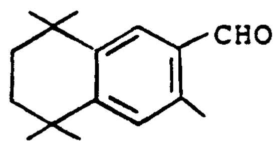

V

aus US-PS 2 800 511 (c). Der Verbindung IV wird in (b) - zu Recht - eine bloss schwache Ambranote zugeschrieben; V weist zwar gemäss (c) eine Moschusnote auf, doch wird sie - unter denselben Bedingungen wie I evaluiert (5%ige alkoholische Lösung) - als staubig, weniger intensiv, mit metallischer Kopfnote beschrieben.

Auch für das Formyl-1,1,3,4,4,6-hexamethyltetralin des Beispiels 2 der DE-OS 1468755 gilt entsprechend diese geringere Intensität, zudem verbunden mit einer organoleptisch deutlich weniger ansprechenden Moschusnote.

Die Verbindungen der Formel I eignen sich aufgrund ihrer natürlichen Geruchsnoten insbesondere zur Modifizierung von bekannten Kompositionen.

Bemerkenswert ist ferner die Art und Weise, wie die Verbindungen I die Geruchsnoten bekannter Kompositionen abrunden und harmonisieren, ohne aber in unangenehmer Weise zu dominieren.

Die Verbindungen I verbinden sich mit zahlreichen bekannten Riechstoffingredientien natürlichen oder synthetischen Ursprungs, wobei die Palette der natürlichen Rohstoffe sowohl leicht- als auch mittel- und schwerflüchtige Komponenten, und diejenige der Synthetika Vertreter aus praktisch allen Stoffklassen umfassen kann, wie dies aus der folgenden Zusammenstellung ersichtlich ist:

- Naturprodukte, wie Baummoos-Absolue, Basilikumöl, Agrumenöle (wie Bergamotteöl, Mandarinenöl, etc.), Mastix-Absolue, Myrtenöl, Palmarosaöl, Patchouliöl, Petitgrainöl, Paraguay, Wermutöl,
- Alkohole, wie Farnesol, Geraniol, Linalool, Nerol, Phenyläthylalkohol, Rhodinol, Zimtalkohol,
- Aldehyde, wie Citral, Helional®, $\alpha$-Hexylzimtaldehyd, Hydroxycitronellal, Lilial® (p-tert.Butyl-$\alpha$-methyldihydrozimtaldehyd), Methylnonylacetaldehyd,
- Ketone, wie Allyljonon, $\alpha$-Jonon, $\beta$-Jonon, Isoraldein (Isomethyl-$\alpha$-ionon), Methyljonon,
- Ester, wie Allyl-phenoxyacetat, Benzyl-salicylat, Cinnamylpropionat, Citronellyl-acetat, Citronellyl-äthoxolat (Citronellyl . O - CO -CO . $OC_2H_5$), Decylacetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat, etc.
- Lactone, wie $\gamma$-Undecalacton,
- verschiedene, in der Parfümerie oft benützte Komponenten, wie Ketonmoschus, Indol, p-Menthan-8-thiol-3-on, Methyleugenol.

Die Verbindungen der Formel I (bzw. deren Gemische) lassen sich in weiten Grenzen einsetzen, die beispielsweise von ca. 0.1 (Detergentien) ca. -20% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 1 und ca. 10%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Gewebeveredler, Tabak, etc.).

Die Verbindungen I können demgemäss bei der Herstellung von Kompositionen und - wie obige Zusammenstellung zeigt -unter Verwendung einer breiten Palette bekannter Riechstoffe bzw. Riechstoffgemische verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2, 7. Auflage, Chapman und Hall, London, 1974 hervorgehend.

Beispiel 1

In einen 4-Halskolben, versehen mit mechanischem Rührer, Tropftrichter, Rückflusskühler und Thermometer gibt man 580 g 70%ige Schwefelsäure. Unter Rühren bei 60 °C wird nun rasch eine Lösung von 308 g 1,1,2,4,4,7-Hexamethyl-1,2,3,4-tetrahydronaphthalin in 500 ml Cyclohexan zugetropft.

Bei dieser Temperatur werden in kleinen Portionen innerhalb einer Viertelstunde 209 g Mangandioxyd zugegeben. Die Temperatur steigt auf 65 °C und wird noch auf 80 °C erhöht, bei welcher Temperatur das Gemisch 4 Stunden gerührt wird. Danach wird heiss filtriert (Büchner-Trichter mit 20 g Celatom FW 50 als Filterhilfsmittel) und mit 400 ml heissem Cyclohexan nachgewaschen. Die vereinigten Filtrate werden mit Wasser und $NaHCO_3$-Lösung neutral gewaschen und am Rotationsverdampfer eingeengt. Es werden 308 g Rohprodukt erhalten, bestehend aus einem 2:1 Gemisch von Ausgangsmaterial und gewünschtem Aldehyd Ib (GC, Carbowax 20 M/220 °C). Die Destillation über eine Goodloe-Kolonne liefert den reinen Aldehyd Ib in 23%iger Ausbeute, Siedepunkt 118 °C/66,66 Pa (0,5 mm Hg), Reinheit (GC) >95%. Umkristallisation aus Aethanol liefert 99%iges Material mit einem Schmelzpunkt von 77,5-78,5 °C.

Beispiel 2

Der Kohlenwasserstoff 1,1,2,4,4-Pentamethyl-1,2,3,4-tetrahydronaphthalin wird in Methylenchlorid vorgelegt und bei -20 °C mit einer äquimolaren Menge $TiCl_4$ versetzt. Unter Rühren wird ebenfalls in äquimolarer Menge 1,1-Dichlordimethyläther zugetropft und die Temperatur innerhalb der folgenden 4 1/2 Stunden auf +30 °C gebracht. Das Gemisch wird anschliessend auf Eis gegossen, mit Aether extrahiert und mit gesättigter NaCl, verdünnter $NaHCO_3$ und gesättigter NaCl neutral gewaschen, über $MgSO_4$ getrocknet und eingeengt und im Vakuum destilliert. Das erhaltene Rohprodukt wird aus Aethanol umkristallisiert und zeigt nun ein nur unwesentlich breiteres Schmelzpunktsintervall als im Falle des Beispiels 1. Gaschromatographisch (Kapillar-kolonne SE 30) kann es von Ib nicht unterschieden werden. Gemäss den NMR-Daten (400 Mhz) handelt es um ein 1:1 Gemisch der Aldehyde Ia und Ib.

Durch mehrfache Kristallisation aus Alkohol wird eine angereicherte Fraktion von Ib (Reinheit 90%) erhalten, welche mit dem Material aus Beispiel 1 identisch ist (GC und NMR). In den Mutterlaugen verbleibt das andere Isomere (Ia), dessen Identität durch NMR-Vergleich mit Ib etabliert werden kann, Reinheit > 90% (NMR). Olfaktisch sind Ia und Ib sehr ähnlich, das heisst praktisch kaum zu unterscheiden und somit für die Anwendung untereinander austauschbar bzw. auch als Gemisch brauchbar.

Beispiel 3

a) Kompositionen mit blumiger, frischer, pudriger Hesperidennote

| | Gewichtsteile |
|---|---|
| Aethylvanillin | 1 |
| Neroline (2-Methoxy-naphthalin) | 2 |
| Galbanumessenz | 2 |
| Vernaldehyde (1-Methyl-4-(4-methylpentyl)-3-cyclohexen carbaldehyd) | 5 |
| Bornylacetat | 5 |
| Cananga-essenz | 10 |
| Sandalore (5-(2,3,3-Trimethyl-cyclopent-3-enyl)3-methyl-pentan-2-ol) | 15 |
| Petitgrain ess. Paraguay | 20 |
| Verdylacetat (Dihydro-nor-dicyclopentadienyl-acetat) | 20 |
| Dimetol (2,6-Dimethyl-heptan-2-ol) | 20 |
| Gewürznelkenblätteröl | 20 |
| Benzylacetat | 30 |
| Coumarin | 30 |
| Terpineol | 30 |
| Methyl-cedryl-keton | 40 |
| Madrox (1-Methyl-1-methoxy-cyclodedecan) | 50 |
| Citronellol | 50 |
| Geraniol | 50 |
| Isoraldéine 70 (Isomethyl-ionone/Iso-α-4-(2,6,6-trimethyl-2-cyclohexen-1-yl)3-methyl-3-buten-2-on) | 100 |
| Benzylsalicylat | 100 |
| Tetrahydrolinalool | 100 |
| p-tert. Butylcyclohexylacetat | 150 |
| Lilial (α-Methyl-3-(p-tert-butylphenyl)-propionaldehyd) | 50 |
| | 900 |

Zugabe von 100 Gewichtsteilen Ib resultiert im Auftreten bemerkenswerter Intensität, Strahlungskraft und Diffusion.

b) Tabak-Leder-Base

| | Gewichtsteile |
|---|---|
| Sellerieessenz | 1 |
| Helichrysium-(Immortellen)resinoid | 1 |
| Kadeöl in DPG | 1 |
| 6-sec-Butylchinolin | 1 |
| Zimtaldehyd | 1 |
| Ambroxane (8α-12-Oxido-13,14,15,16-tetranorlabdan) | 1 |
| Eugenol | 1 |
| Aethylvanillin | 5 |
| Flouve absolue | 5 |
| Phenylpropylalkohol | 20 |
| Coumarin | 30 |
| Sandalore (5-(2,2,3-Trimethyl-cyclopent-3-enyl)-3-methyl pentan-2-ol) | 30 |
| Zedernholzessenz | 50 |
| Weihrauchresinoid | 50 |
| β-Ionon | 50 |
| Linalool | 50 |
| Linalylacetat | 100 |
| Amylsalicylat | 100 |
| Patchouliessenz | 100 |
| Nonanylacetat | 100 |
| Isoraldéine 70 (Isomethyl-ionon und iso-α-4-(2,6,6-Trimethyl-2-cyclohexen-1-yl)-3-methyl-3-buten-2-on) | 200 |
| Gardenol (Methylphenyl-carbinylacetat) | 3 |
| | 900 |

Zugabe von 100 Teilen Aldehyd Ib resultiert im Auftreten einer ausgeprägten Tabaknote, die ausgesprochen reich, natürlich und ausgeglichen harmoisch wirkt.

c) Eau de Toilette mit den Noten: Citrus, blumig, holzig, frisch

| | Gewichtsteile |
|---|---|
| $C_{10}$-Aldehyd | 2 |
| $C_{11}$-Aldehyd | 2 |
| Vanillin | 2 |
| Pêche pure (γ-Undecalacton) | 2 |
| Ciste labdanum-resinoid | 4 |
| Neroli ess. bigarade | 4 |
| Gardenol (Methylphenylcarbinylacetat) | 6 |
| Isobutyl-chinolin 10% in DPG | 6 |
| Galbanum-essenz | 6 |
| Coumarin | 10 |
| Ylang-ylang-essenz | 10 |
| Lilial (α-Methyl-β-(p-tert. butyl-phenyl)-propionaldehyd) | 20 |
| Lemarome N (3,7-Dimethyl-2,6-octadienal = Citral synthet.) | 20 |
| Geranium-essenz Bourbon | 20 |
| Bois de Santal-essenz | 20 |
| Dipropylenglykol | 36 |
| Citronellol extra | 40 |
| Vetivenylacetat Haiti | 40 |
| Mousse de chêne abs. 50% in Benzylbenzoat | 40 |
| Lavandin-essenz | 40 |
| Patchouli-essenz | 60 |
| Madrox (1-Methyl-1-methoxy-cyclododecan) | 60 |
| Isoraldéine (4-(2,6,6-Trimethyl-2-cyclohexen-1-yl)-3-methyl-3-buten-2-on/Methyl-ionon) | 100 |
| Hydroxycitronellal | 100 |
| α-Hexylzimtaldehyd | 100 |
| Citronen-essenz | 100 |
| Bergamotte-essenz | 100 |
| | 950 |

Durch Zugabe von 50 Teilen des Aldehyds Ib resultiert eine Komposition mit ausgeprägtem Frischeeffekt, insbesondere bedingt durch die starke Diffusion der Citrusnoten. Ebenfalls angenehm treten die Holznoten hervor. Die Komposition wirkt - auch im Fond - ausgewogen, voll.

**Patentansprüche**

**1.** Verbindungen der Formel

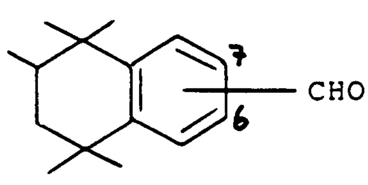

I

**2.** 1,1,2,4,4-Pentamethyl-6-formyl-1,2,3,4-tetrahydronaphthalin.

**3.** 1,1,2,4,4-Pentamethyl-7-formyl-1,2,3,4-tetrahydronaphthalin.

**4.** Riechstoffkomposition, gekennzeichnet durch einen Gehalt an einer Verbindung der Formel

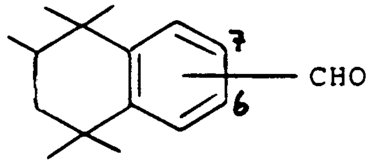

I

5. Verfahren zur Herstellung der Verbindungen der Formel

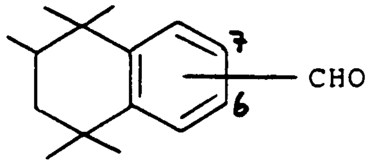

I

dadurch gekennzeichnet, dass man eine Verbindung der Formel

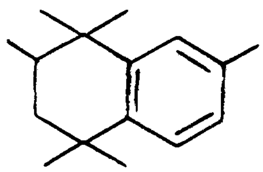

II

oxydiert, oder dass man eine Verbindung der Formel

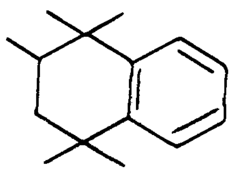

III

formyliert.

6. Verwendung der Verbindungen der Formel

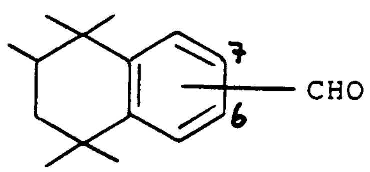

I

als Riechstoffe.

**Claims**

1. Compounds of the formula

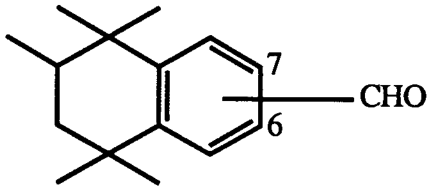

I

**2.** 1,1,2,4,4-Pentamethyl-6-formyl-1,2,3,4-tetrahydronaphthalene.

**3.** 1,1,2,4,4-Pentamethyl-7-formyl-1,2,3,4-tetrahydronaphthalene.

**4.** An odorant composition, characterized in that it contains a compound of the formula

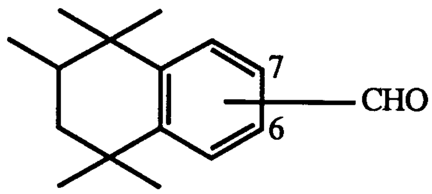

I

**5.** A process for the manufacture of the compounds of the formula

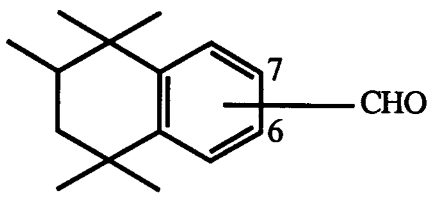

I

characterized by oxidizing a compound of the formula

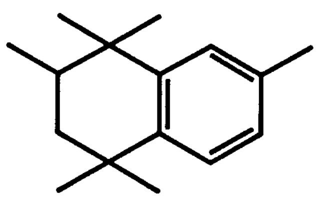

II

or formylating a compound of the formula

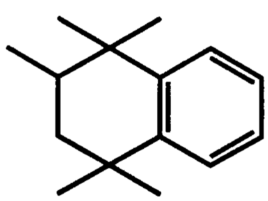

III

**6.** The use of the compounds of the formula

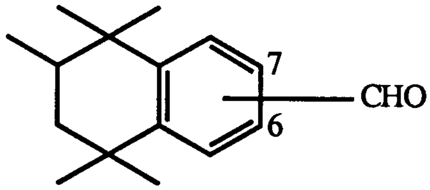 I

as odorants.

## Revendications

1. Composés de formule :

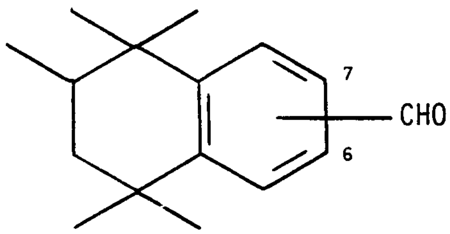 I

2. 1,1,2,4,4-pentaméthyl-6-formyl-1,2,3,4-tétrahydronaphtalène.

3. 1,1,2,4,4-pentaméthyl-7-formyl-1,2,3,4-tétrahydronaphtalène.

4. Composition odorante caractérisée en ce qu'elle contient un composé de formule :

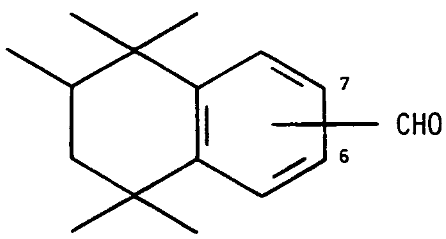 I

5. Procédé de préparation des composés de formule :

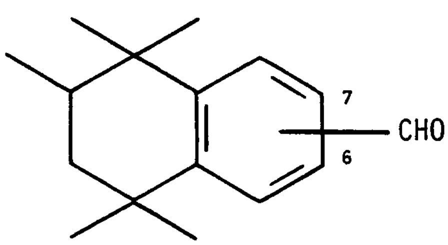 I

caractérisé en ce qu'on oxyde un composé de formule :

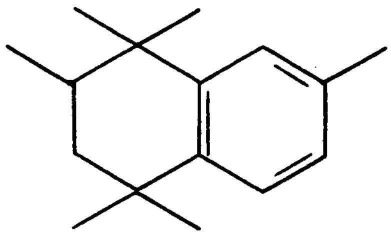

II

ou en ce qu'on formyle un composé de formule :

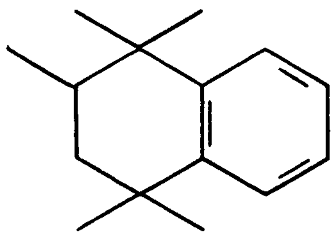

III

**6.** Application des composés de formule :

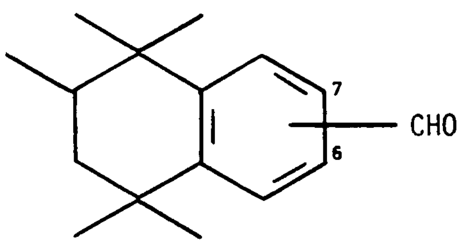

I

comme parfums.